**Europäisches Patentamt**

**European Patent Office**

**Office européen des brevets**

(19)

(11) Veröffentlichungsnummer: **0 147 459**
**B1**

(12) # EUROPÄISCHE PATENTSCHRIFT

(45) Veröffentlichungstag der Patentschrift:
02.09.87

(21) Anmeldenummer: 84902700.8

(22) Anmeldetag: 27.06.84

(86) Internationale Anmeldenummer:
PCT/EP 84/00194

(87) Internationale Veröffentlichungsnummer:
WO 85/00097 (17.01.85 Gazette 85/02)

(51) Int. Cl.⁴: **A 61 B 1/00**, A 61 M 25/00

(54) **MEDIZINISCHE GERÄTE MIT EINFÜHRUNGSHILFE.**

(30) Priorität: 28.06.83 CH 3526/83
12.08.83 DE 3329176

(43) Veröffentlichungstag der Anmeldung:
10.07.85 Patentblatt 85/28

(45) Bekanntmachung des Hinweises auf die Patenterteilung:
02.09.87 Patentblatt 87/36

(84) Benannte Vertragsstaaten:
AT BE CH DE FR GB LI LU NL SE

(56) Entgegenhaltungen:
DE-A-2 823 025
FR-A-1 456 623
FR-E-90 209
US-A-4 321 915

(73) Patentinhaber: Sterimed Gesellschaft für
medizinischen Bedarf mbH, Fasanerieweg 15- 17
Postfach 215, D-6600 Saarbrücken 3 (DE)

(72) Erfinder: KRAMANN, Bernhard, Preysingstrasse 11,
D-8000 München 80 (DE)

(74) Vertreter: Suchy, Herbert, Dr., Byk Gulden
Lomberg Chemische Fabrik GmbH Byk- Gulden-
Strasse 2 Postfach 6500, D-7750 Konstanz (DE)

EP 0 147 459 B1

## Beschreibung

Die Erfindung betrifft ein längliches flexibles medizinisches Gerät zum Untersuchen oder Behandeln von Körperhöhlen, insbesondere Endoskope, mit Einführungshilfsvorrichtung nach dem Prinzip des sich ausstülpenden Schlauchgebildes.

Das Einführen von länglichen flexiblen medizinischen Untersuchungsgeräten, wie z. B. Endoskope, in Körperhöhlen, die einen gewundenen Verlauf aufweisen, wie z. B. den Darm oder den Ösophagus, bereitet Schwierigkeiten und ist nicht ohne Risiko für den Patienten. Es gibt deshalb zahlreiche Vorschläge, wie dieses Einführen verbessert werden kann.

In der DE-A- 28 23 025 wird eine Vorrichtung zum Einführen eines Endoskopes zur Darmdiagnostik beschrieben, die aus einem rohrförmigen Gehäuse besteht, an dessen distraler Öffnung ein dünnwandiger Schlauch angebracht ist. Dieser Schlauch ist in das Innere des Gehäuses gestülpt und ist an seinem anderen Ende mit dem distalen Ende eines Endoskopes verbunden. Durch Beaufschlagung des Gehäuses mit einem Druckmedium stülpt sich der Schlauch aus dem Gehäuse in die Körperöffnung, wobei das Endoskop nachgezogen wird. Der Schlauch kleidet mit fortschreitender Ausstülpung die Körperhöhle aus, so daß das Endoskop biem Eindringen in die Körperhöhle mit deren Wandung nicht in Berührung kommt. Dieses an sich für die Wandung der Körperhöhle sehr schonende Verfahren hat den Nachteil, daß der Untersucher durch das Endoskop erst dann die Wandung der Körperhöhle inspizieren kann, wenn der Schlauch völlig ausgestülpt ist.

Diesen Nachteil umgeht die in der US-A-4 321 915 beschriebene sehr ähnliche Vorrichtung dadurch, daß der Schlauch nicht am distalen Ende des Endoskopes befestigt ist, sondern an einer dichtend auf dem Endoskop beweglichen Hülse. Da das Endoskop bei Druckbeaufschlagung des Gehäuses sich in der Körperhöhle doppelt so schnell vorwärtsbewegt wie die Auskleidung der Wand der Körperhöhle durch den sich umstülpenden Schlauch erfolgt, wird abwechselnd Druck und Unterdruck angelegt und während der Unterdruckphasen das Endoskop relativ zum Schauch ein Stück zurückgezogen.

Aufgabe der vorliegenden Erfindung ist es, ein längliches flexibles medizinisches Gerät mit einer Einführungshilfsvorrichtung nach dem Prinzip des ausstülpbaren Schlauches zur Verfügung zu stellen, das konstruktiv und in der Anwendung einfacher ist, als diejenigen nach dem Stand der Technik. Es wird dabei von der Erkenntnis Gebrauch gemacht, daß es nicht notwendig ist, die Einführung des medizinischen Geräts während des gesamten Vorschubes durch den sich ausstülpenden Schlauch zu unterstützen; es genügt vielmehr, das medizinische Gerät bei unproblematischen Passagen der Körperhöhlung auf konventionelle Weise vorzuschieben und nur bei problematischen Passagen vom Prinzip des sich ausstülpenden Schlauches Gebrauch zu machen.

Gelöst wird die Aufgabe dadurch, daß die Einführungshilfsvorrichtung aus einem beidseitig offenen Rohr mit seitlichem Druckanschlußstutzen und einem durch das Rohrverlaufendem flexiblen ausstülpbaren Schlauchgebilde besteht, dessen beide Enden mit jeweils einem Ende des Rohres verbunden sind, wobei das medizinische Gerät innerhalb des Schlauchgebildes durch das Rohr läuft und das Schlauchgebilde im Bereich des distalen Endes des medizinischen Geräts in mehreren Doppellagen aufgefaltet ist.

Die Anwendung des erfindungsgemäßen Gegenstandes erfolgt nun so, daß das medizinische Gerät an der Körperöffnung der Körperhöhle, in die die Einführung erfolgen soll, angesetzt wird und solange auf konventionelle Weise vorgeschoben wird bis sich durch Erhöhung des Widerstandes eine Krümmung oder ein sonstiges Hindernis bemerkbar macht. Nunmehr wird das Einführungshilfsgerät mit Druck beaufschlagt und das medizinische Gerät weiter vorgeschoben, wobei sich die erste Lage der mehrlagigen Auffaltung des Schlauchgebildes im Bereich des distalen Endes des medizinischen Geräts entfaltet, d.h. sich in die Körperhöhle ausstülpt. Das medizinische Gerät läßt sich dabei leicht weiter einführen und zwar mit doppelter Geschwindigkeit wie die Ausstülpungsfront der sich ausstülpenden Lage des Schlauchgebildes vordringt. Neben der leichteren Vorschiebbarkeit des medizinischen Geräts macht sich das Einstülpen des Schlauchgebildes für den Untersucher dadurch bemerkbar, daß sich das proximale Ende des medizinischen Gerätes auf das proximale Ende der Einführungshilfseinrichtung zubewegt. Nach Überwindung des Hindernisses wird das medizinische Gerät wieder auf konventionelle Weise, also unter Reibung an der Wand der Körperhöhle, weitergeschoben bis sich ein weiteres Hindernis bemerkbar macht. Auch dieses und alle weiteren Hindernisse werden überwunden, indem die Einführungshilfseinrichtung mit einem Druckmedium beaufschlagt wird und das medizinische Gerät mit dem sich einstülpenden Schlauchgebilde verschoben wird. Sollte hierbei das proximale Ende des medizinischen Geräts dem proximalen Ende der Einführungshilfsvorrichtung so nahe kommen, daß einweiteres Ausstülpen des Schlauchgebildes unter Druck nicht mehr möglich ist, so kann die Einführungshilfsvorrichtung in Richtung auf den Patienten geschoben werden und die dabei freiwerdende Länge des Schlauchgebildes auf das distale Ende der Einführungshilfsvorrichtung geschoben werden, nötigenfalls in mehreren Lagen, und dort gewünschtenfalls z. B. mittels passender Klammern fixiert werden.

Der rohrförmige Teil der Einführungshilfsvorrichtung samt Druckanschlußstutzen wird aus Metall oder soweit zur Vermeidung der Resterilisierung an ein Einmalgerät gedacht ist, aus Kunststoff, wie beispielsweise Polyamid oder Polyethylen, hergestellt. Das Schlauchgebilde wird aus Materialien hergestellt, die für solche dünnwandigen flexiblen Gebilde üblich sind. Als besonders zweckmäßig erweisen sich Materialien, die etwas durchlässig für das verwendete flüssige Druckmedium sind, wie beispielsweise Celluloseacetat, weil dadurch das Druckmedium gleichzeitig als Schmiermittel wirken kann. Die Befestigung der Enden des Schlauchgebildes an dem Rohr der Einführungshilfsvorrichtung geschieht auf übliche Weise durch Verkleben oder Verschweißen oder andere geeignete Befestigungsmittel, wie z. B. einen Schrumpfschlauch.

Als Druckmedium kommen insbesondere Flüssigkeiten in Frage. Bei Verwendung von wäßrigem Medium ist ein Zusatz von z. B. Glycerin oder Paraffinum perliquidum zur Verbesserung der Gleiteigenschaften zweckmäßig.

Unter einem länglichen medizinischen Gerät zum Untersuchen oder Behandeln von Körperhöhlen werden insbesondere Endoskope, Probeentnahmegeräte und Spülvorrichtungen zur rektalen, oralen oder nasalen Einführung verstanden. Es versteht sich von selbst, daß die Erfindung auch auf anderen Gebieten der Technik nutzbringend angewendet werden kann, wo das Problem des Zuganges für Endoskope und Spülvorrichtungen in nichtlinearen Höhlen besteht.

Nachstehend wird die Erfindungsanhand von Ausführungsbeispielen näher erläutert.

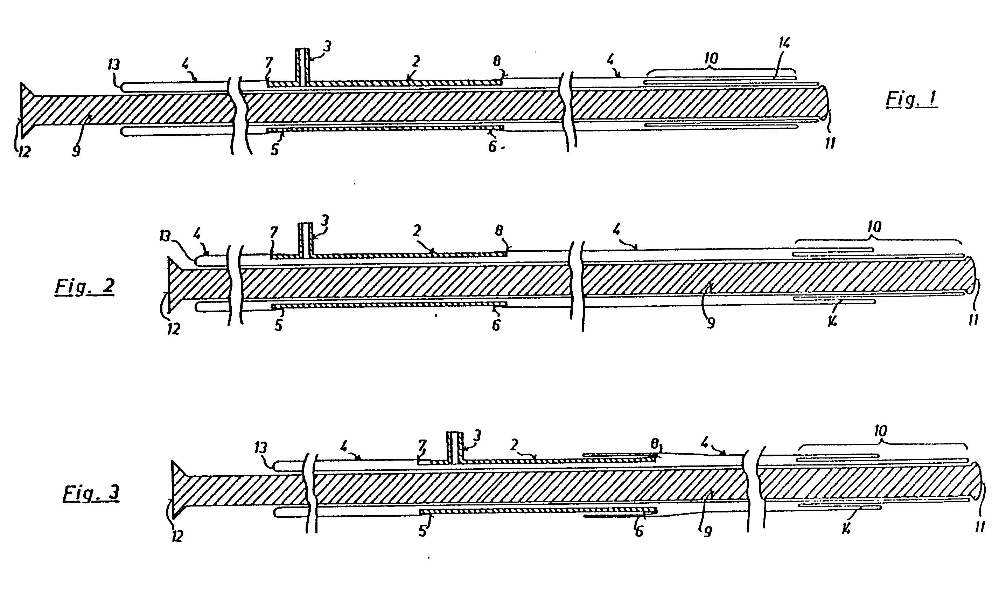

Fig. 1 bis 3 zeigen den Erfindungsgegenstand schematisch in verschiedenen Phasen seiner Anwendung.

Fig. 1 zeigt schematisch ein medizinisches Gerät 9, das z. B. ein Endoskop zur Untersuchung des Dickdarms sein kann, in der Ausgangsstellung. Das Gerät 9 ist durch das durch das Rohr 2 laufende Schlauchgebilde 4 geschoben. Im distalen Bereich 10 des Gerätes 9 ist das Schlauchgebilde 4 in mehreren Doppellagen aufgebracht, so daß das distale Ende 11 frei bleibt. Das Schlauchgebilde 4 liegt an dem Gerät 9 gerade so straff an, daß es beim Einführen in die Körperhöhle nicht auf dem Gerät 9 zurückgeschoben werden kann. Dieses Anliegen des Schlauchgebildes 4 auf dem Gerät 9 ist aus Gründen der Übersichtlichkeit zeichnerisch nicht dargestellt.

Das proximale Ende 12 des Gerätes 9 ragt aus dem Schlauchgebilde 4 heraus, da beim Einschieben des Gerätes 9 unter Druckbeaufschlagung durch Druckanschlußstutzen 3 sich das proximale Ende 12 des Gerätes 9 auf die Einstülpfront 13 des Schlauchgebildes 4 zubewegt. Beim Einschieben des Gerätes 9 in das Schlauchgebilde 4 bewegt sich nämlich dessen Einstülpfront 13 nur halb so schnell wie das Gerät 9.

Fig. 2 zeigt das Gerät 9 schematisch in einer Stellung, wo die äußerste Doppellage 14 des Schlauchgebildes 4 durch Vorschieben des Geräts 9 unter Druckbeaufschlagung durch den Druckanschlußstutzen 3 teilweise ausgestülpt ist. Das proximale Ende 12 des Gerätes 9 bewegt sich dabei in Richtung auf das Rohr 2. Um weiteren Raum zum Vorschieben des Geräts 9 zu bekommen, kann das Rohr 2 in Richtung distal gegenüber dem Gerät 9 verschoben werden.

Fig 3 zeigt das Gerät 9 schematisch in einer Stellung, in der das Rohr 2 gegenüber dem Gerät 9 nach distal verschoben ist. Die dabei auf der distalen Seite des Rohrs freiwerdende Länge des Schlauchgebildes 4 wird jeweils auf das Rohr 2 aufgestülpt. Je nach Länge des aufzustülpenden Schlauchgebildes 4 wird in mehreren Lagen aufgestülpt. Der auf das Rohr 2 aufgestülpte Teil des Schlauchgebildes wird gewünschtenfalls durch geeignete Klemmen (in der Fig. nicht dargestellt) fixiert. Um den auf das Rohr 2 aufgestülpten Teil des Schlauchgebildes 4 zusätzlich gegen ein Abrutschen zu sichern, kann die Außenseite des Rohres 2 beispielsweise quergeriffelt sein.

Im Falle eines Endoskopes zur Untersuchung des Dickdarms hat das Beispielsweise eine Länge von rund 2 m. Der im distalen Bereich 10 des Gerätes 9 in mehreren, vorzugsweise zwei bis drei Doppellagen aufgebrachte Teil des Schlauchgebildes 4 hat eine Länge von ungefähr 20 bis 40 cm. Die Länge des im distalen Bereich 10 des Gerätes 9 in Doppellagen aufgebrachten Teils des Schlauchgebildes 4 soll mindestens der Summe der Längen der beim Einführen zu erwartenden schwierigen Passagen entsprechen. Das proximale Ende 12 des Geräts soll aus dem proximalen Ende 7 des Rohres 2 um ein Stück herausragen, das der Summe der Längen der zu erwartenden schwierigen Passagen entspricht. Es ist zweckmäßig, in der Ausgangsstellung den Abstand der Einstülpfront 13 vom proximalen Ende 7 des Rohres 2 maximal auf die Hälfte des Abstandes zwischen dem proximalen Ende 12 des Gerätes 9 und dem proximalen Ende 7 des Rohres 2 einzustellen. Das Rohr 2 weist eine Länge von etwa 20 cm auf. Sein innerer Durchmesser ist etwas größer als der Außendurchmesser des Gerätes 9 am distalen Ende 11.

## Patentansprüche

1. Längliches medizinisches Gerät zum Untersuchen oder Behandeln von Körperhöhlen, insbesondere Endoskop, mit Einführungshilfsvorrichtung nach dem Prinzip eines sich ausstülpenden Schlauchgebildes (4), dadurch gekennzeichnet, daß die Einführungshilfsvorrichtung (1) aus einem beidseitig offenen Rohr (2) mit seitlichem Druckanschlußstutzen (3) und einem durch das Rohr (2) verlaufendem flexiblen ausstülpbaren Schlauchgebilde (4) besteht, dessen beide Enden (5, 6) mit jeweils einem Ende (7, 8) des Rohres (2) verbunden sind, wobei das medizinische Gerät (9) innerhalb des Schlauchgebildes (4) durch das Rohr (2) läuft und das Schlauchgebilde (4) im Bereich (10) des distalen Endes (11) des medizinischen Gerätes (9) in mehreren Doppellagen aufgefaltet ist.

## Claim

1. An elongated medical instrument for the examination or treatment of body cavities, in particular endoscopes, with a device to assist introduction by the principle of the tubular structure which becomes everted, comprises the device to assist introduction (1) being composed of a pipe (2) which is open at both ends and has pressure connectors (3) on the sides, and is composed of a flexible, eversible tubular structure (4) running through the pipe (2), the two ends (5, 6) of the tubular structure each being connected to one end (7, 8) of the pipe (2), the medical instrument (9) running through the pipe (2) inside the tubular structure (4), and the tubular structure (4) being folded in several double-layers in the region (10) of the distal end (11) of the medical instrument (9).

## Revendication

1. Appareil médical de forme allongée pour l'examen ou le traitement des cavités corporelles, en particulier endoscope, muni d'un dispositif d'aide d'introduction selon le principe d'une structure de tuyau (4) extroversible, caractérisé en ce que le dispositif d'aide d'introduction (1) est constitué par un tube (2) ouvert des deux côtés et pourvu de tubulures de raccord de pression (3) laterales et par une structure de tuyau (4) flexible extroversible qui traverse le tube (2) et dont les deux extrémités (5, 6) sont chacune reliées à une extrémité (7, 8) du tube (2), l'appareil médical (9) passant dans le tube (2) à l'intérieur de la structure de tuyau (4) et la structure de tuyau (4) étant repliée à superposition en plusieurs doubles couches dans la zone (10) de l'extrémité distale (11) de l'appareil médical (9).

_Fig. 1_

_Fig. 2_

_Fig. 3_

0 147 459